# EUROPEAN PATENT APPLICATION

(11) **EP 0 952 384 A2**
(43) Date of publication of application: **27.10.1999**
(21) Application number: 98600020.6
(22) Date of filing: 15.12.1998
(51) Int. Cl.: F16P 1/06

(54) **Protective mask with movable safety eye plate**

(30) Priority: 15.12.1997 GR 97200244
(71) Applicant: Tovstiadis, George, 185 45 Piraeus (GR)
(72) Inventor: Tovstiadis, George, 185 45 Piraeus (GR)

(57) **Abstract**

The protective mask is intended for works where a face and eye protection of the operator against injuries is needed, particularly welding, to protect the eyes and the skin of the operator against light and mechanic effects, as well as from molten metal splashes during the operation process. A characteristic feature is that the position of the eye plate (3) is adjusted by mandible movements. By moving the mandible a device for turning (6) adjusts the eye plate (3) in the required position so that each following action of the mandible places the plate (3) in the contrary position, closing it for eye protection or opening it for free vision. The mask sets completely free the hands of the operator while working. The protective mask comprises a protection shell (1) with observation cut-outs (2) covered by the safety eye plate (3) attached to an axis (4) having an eccentric lever (5) for turning up on one of its ends, being the axis (4) connected to the shell (1). It also coprises a device (6) for turning the safety eye plate (3) having an under-mandible-lever (7) operated by the mandible of the worker. The device (6) is connected to the axis (4). The mask comprises also a device for fixing to the head of the operator connected to the shell (1), provided with clamps (9) supported by the concha cartilage, as well as adjustable rest (12), connected to the shell (1) in the area of the operator's nostrils. A fixing clip (13) with holders (14) for fixing to the upper part of the nose could be additionally provided, being the holders (14) connected with a spring (16) for keeping tight. The shell (1) could be made by at least two parts: upper (20) and lower (21), flexibly connected to each other so that a mutual adjustment in the desired position according to the face size could be made.

## Description

### FIELD OF THE INVENTION

The invention relates to a protective mask with movable safety eye plate used to protect the face of an operator against injuries, and particularly during welding, to protect the eyes and the skin of the operator against light and mechanic effects, as well as from molten metal splashes during the operation process.

### BACKGROUND OF THE INVENTION

A protective welding mask from DE 1961674 of 1 December 1969 is known in the art including a solid shell with observation cut-outs and device for fixing the mask to the operator's head. The mask known in the art further comprises a movable safety light filter plate connected to the shell by guides placed laterally to the observation gaps, said guides enabling the plate slide. A second safety plate fixed firmly to the shell is provided, too. There is a device connected to the movable light filter plate for its adjustment along the guides by the operator moving its mandible, comprising this device a mandible lever. The device for fixing the mask on the operator's head is an embracing band, fixed to the mask shield, with adjustable tightening. The advantage of this structure is that the adjustment of the position of the movable light filter plate could be effected by moving the operator his mandible without using his hands. Thus leaving partial free his hands for performing other movements needed during the operation of welding. However the constant wearing of the face mask for different periods of time and breaks in the arch welding is burdened by the miserable condition of the operator: he should remain with an open mouth all the time. The execution of any fitting and preliminary works requiring precision are also troubled as the scope of vision of the working place is affected by the second inner plate fixed to the shell, that is exposed to splashes and smoke. The continuous and frequent action of the mandible on the mask causes its displacement, which increases the requirements to its fixing to the head. The mask known in the art is fixed to the head by seizing as with a ring the mobile scalp of the head without using the parts fixed to the skull bone. As far as a part of the mandible displacement is on account of the displacement of the mask, the resulting displacement of the movable safety plate is making it worse. Another problem is that the size of the mask is calculated for a large face. In any other case the operator will use a mask of a larger compared to his face size. So, the lower end of the mask that projects under the chin of the operator will limit the freedom of movements to his head.

### SUMMARY OF THE INVENTION

A problem solved by the invention is that the mask sets completely free the hands of the worker by allowing a long use without removing it and does not disturb the movements of the operator.

Another problem that is solved is that the mask offered provides a wider scope of vision to the operator at a drawn down eye plate that is close to the natural human view. The vision quality is not getting worse compared to the natural one, because in this position there are no intermediate devices between the eyes of the worker and the observed object.

Another problem solved is the stable attachment of the mask to the face by using the immovable with regards to the skull parts of the head.

Next problem solved with the utility model is that the mask does not obstruct the freedom of movement of the worker's head.

Next problem solved is that the mask may be of universal application where a protection of the human face and eyes against harmful lights and mechanic injuries is sought.

Next problem that is solved with the mask is the prevention of a possible dazzling of the welder by the electric arch by using eye protection shield of different shadowing in its upper and lower parts.

Last but not least the mask is for technological manufacture and requires not expensive and scarce materials.

These and other problems are solved by the protective mask comprising a protection shell with observation cut-outs covered by a safety eye plate. This plate is fixed to an axis having eccentric lever for turning up on one of its ends, being the rod connected to the shell. It also comprises a device for rotating the safety eye plate comprising under-mandible-lever operated by the mandible of the worker. There is a fixing element on the axix for limiting its end position. The mask comprises also a device for fixing to the head of the operator connected to the shell. The possibility to turn the plate to an end position provides a good vision when needed with a sole mandible movement, setting free the hands of the worker. This allows a continuous work without interrupting the main process, remaining the mask on the operator's face.

In one embodiment the shell of the safety mask has an anatomic shape following the characteristic outline of the projecting parts of the human face. It may be made of at least two parts: upper and lower, flexibly connected so that a mutual adjustment in the desired position according to the face size could be made. The anatomic form of the mask makes it possible approach the cut-outs in the mask shell near to the eyes of the worker. This widens the scope of vision of the mask, and being the eye plate up, the vision scope is near to the natural one. The vertical size could be adjusted in accordance with the face size, the lower part of the mask not getting below the chin of the worker. That is why the free movements of the operator's head are not limited at all.

In other embodiment of the invention, the device for fixing to the head of the operator comprises clamps supported by the concha cartilage. The clamps are connected to both sides of the shell by screw connectors through which the shell may be fixed tilted for convenience. The clamps are connected each other through an elastic flexible joint for drawing the mask to the operator's face. The mask further comprises an adjustable rest attached to the shell in the area of the nostrils. The rest is a part of the device for fixing to the head. A fixing clip with holders could be additionally provided fixed to the upper part of the nose, the holders being connected to springs for keeping tight. This way of fixing the unit provides a safety and comfortable way for fixing the mask to the head of the worker.

### DESCRIPTION OF THE FIGURES ATTACHED

Figure 1 is a view of the mask according to the invention.
Figure 2 is a lateral view of the mask of fig. 1.
Figure 3 is an inner view of the mask of fig. 1.
Figure 4 is a zoom view of item I of fig. 3 of the device for turning up and down the safety eye plate.
Figure 5 is and A-A section of fig. 4.
Figure 6 is a zoomed partial B-B section of fig. 3.
Figure 7 is an inner view of the mask in one embodiment of the device for turning the eye plate.
Figure 8 is a partial inner view of the mask with a fixing clip.
Figure 9 is a C-C section of fig. 8.
Figure 10 is a D-D section of fig. 8.

### DETAILED DESCRIPTION OF THE INVENTION

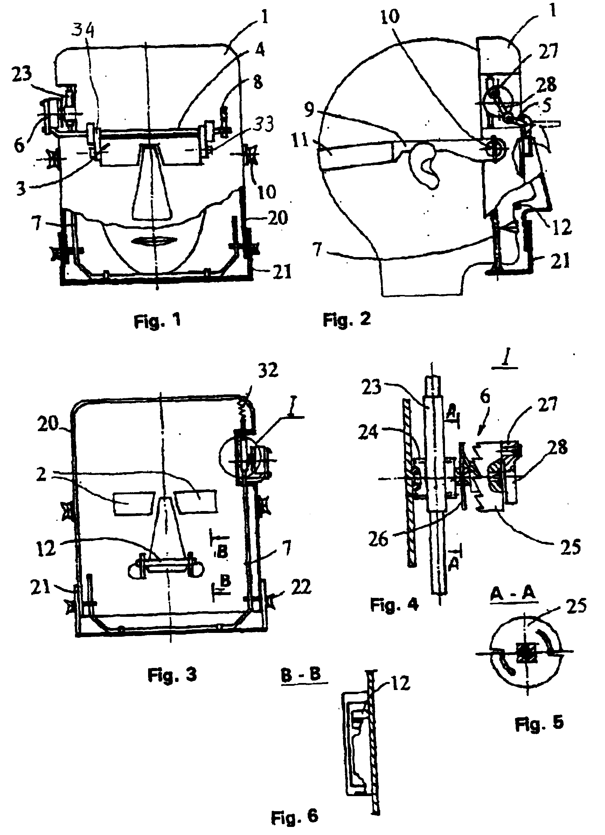

On figures 1-3 is shown the mask according to the invention. A protective mask comprising a protection shell 1 with two observation cut-outs 2 covered by a safety eye plate 3 covering the cut-outs 2. The shell 1 may be made of plastics. The plate 3 is connected demountable to an axis 4 having eccentric lever 5 on one of its ends for turning up and down, being the axis 4 connected to the shell 1. One embodiment of the mask is shown on fig. 1 wherein the plate 3 is connected by screws 33 to fixed to the axis 4 supports 34. The possibility to draw down the plate 3 easily makes the mask of universal application for it could be easily adapted for different use, i.e. for arch electric welding, sharpening of tools, gas welding, etc. The plate 3 is secured against a relative displacement around the axis 4 so that when the axis 4 is rotated it is rotated too. The mask may be provided with three replaceable safety plates 3: black for electric welding, obscured for gas welding and transparent for works wherein face and eye protection against mechanic injuries is needed. The plate 3 used in electric welding could be performed in a way so that its lower part, under the eye level is absolutely obscured, and its upper port over the eye level is partially obscured. This will help the welder see the working site through the upper part of the plate 3 when no electric arch is present and without drawing up the whole plate. This prevents the welder from blinding as a result of an instant electric arch connection.

The device 6 for turning the eye plate 3 is connected to the eccentric lever 5 and comprises a under-mandible-lever 7 operated by the mandible of the worker. A fixture 8 for setting the axis 4 in an end position is attached to the shell. In this embodiment the fixture 8 is a plane spring clammered to the end of the axis 4, the end having a square section. The mask further comprises a device for fixing to the head of the operator provided with two clamps 9 supported by the concha cartilage. Each clamp 9 is connected to one of the sides of the shell 1 by screw connectors 10. By tightening and untightening the connectors 10 the shell 1 may be accommodated in an inclined position for comfortable work with regards to the clamps 9, and, respectively, with regards to the face plane of the worker. Both clamps 9 are connected each other by elastic and flexible joint 11 for tightening the mask to the operator's face.

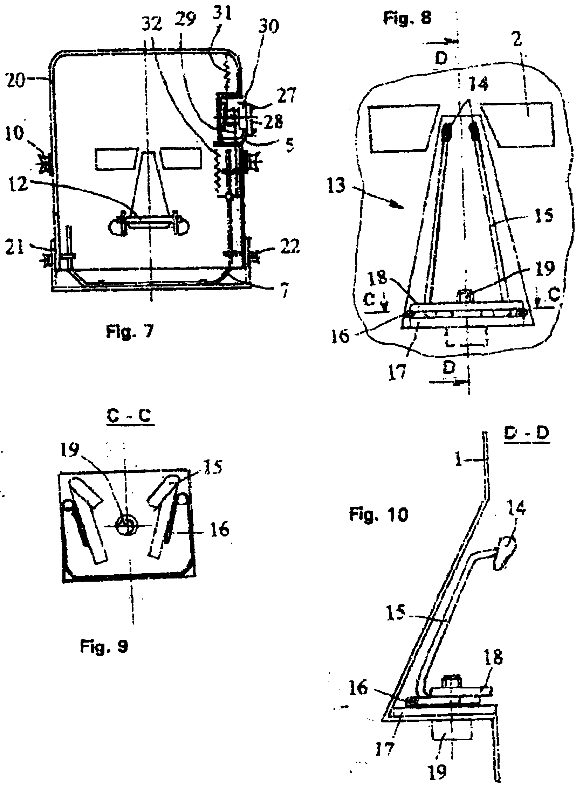

The mask of the embodiment shown on figures 2, 3 and 6 comprises an adjustable rest 12 which is part of the fixing device. The rest 12 is attached to the shell 1 in the area under the nostrils of the operator. On figures 8, 9 and 10 is shown a version of additionally installed fixing clip 13 with holders 14 for attachment to the upper part of the nose. The holders 14 are placed at the end of supports 15, being their first ends connected to springs 16. The springs 16 and the second ends of the supports 15 are placed into a clamp with stationary base 17 and upper tightening part 18, connected by a screw 19. Thus, by untightening the screw 19 the upper part 18 releases the ends of the supports 15 that, under the action of the springs 16 rotate and displace the holders 14 to the nose of the operator. Then the base 17 and the part 18 are tightened again with the screw 19, fixing the holders 14 in this position.

The shell of the mask, according to the embodiments above, has an anatomic shape following the characteristic outline of the projecting parts of the human face. In this case it is made of two parts: upper 20 and lower 21, connected by a screw 22 mounted to the part 20, same could be displaced and tightened in a slit made in the other part 21. That is how the two parts 20 and 21 are fixed in the desired position according to the size of the operator's face.

One embodiment of the device 6 for moving the safety eye plate 3 through the axis 4 is shown on figs 4 and 5. In this case the under-mandible-lever 7 is elastically attached to the shell 1 by means of a spring 32. A rack 23 cogged to a gear 24 with a one-side connection to the shell 1 is placed on it. A ratchet wheel 25 and a disk 26 connected to the gear wheel 24 are placed on the axle of the gear wheel 24. The ratchet wheel 25 has an eccentric pin 27 connected to a rocker 28 of a toggle-rocker device, said rocker 28 connected to the eccentric lever 5 of the axis 4 by hinge joint.

The device 6 that operated the eye plate 3 may be performed as shown on fig. 7. In this embodiment one of the ends of a flexible thread 29 freely wound on a drum 30 connected to the shell 1, is fixed to the under-mandible-lever 7. The other end of the thread 29 is elastically attached to the shell 1, in this case by means of an extension spring 31. The under-mandible-lever 7 is connected to the shell 1 by means of a spring 32. The drum 30 has a similar to the above eccentric pin 27 to which the rocker 28 of the above device is connected, being the rocker 28 connected also to the eccentric lever 5 of the axis 4. In this case, by displacing the under-mandible-lever 7 downwards, the thread 29 runs the drum 30 that through the eccentric pin 27 and the rocker 28 operates the eccentric lever 5 of the axis 4. The plate 3 connected to the xis 4 is turned into an end position where the fixture 8 holds it in that position. Then the worker closes his mouth and the under-mandible-lever 7 is displaced by the spring 32 into its initial position. Thus the end of the thread 29 hangs and by means of the spring 31 it slides freely along the drum surface 30 to return in its initial position. Thus the thread 29 wound around the drum 30 combined with the reverse spring 31 forms an advancing coupling.

The embodiments described are for illustration only and do not limit the invention idea, which is determined solely by the scope of the attached claims.

## Claims

1. A protective mask with movable safety eye plate comprising a protection shell with observation cut-outs and device for fixing to the head of the worker connected to the shell, further comprising a safety eye plate covering the cut-outs, movable connected to the shell, as well as a device for turning the eye plate up and down comprising an under-mandible-lever operated by the mandible of the worker, characterised by that the safety eye plate (3) is mounted to an axis (4) having eccentric lever (5) for turning up and down on one of its edges, being the axis (4) hinged to the shell (1), and the device (6) for turning the plate (3) is of the type transforming the reciprocal motion into a rotary motion and is connected to the eccentric lever (5) of the axis (4), where the axis (4) has a fixture (8) for limiting its end position.

2. A protective mask according to claim 1, characterised by that the device (6) for turning the eye plate (3) comprising a rack (23) operated by the under-mandible-lever (7) hanged by means of a spring (32) to the shell (1), further comprising a gear (24) connected one side to the shell (1) and cogged to the rack (23), further comprising a ratchet coupling with ratchet wheel (25) and a disk (26) connected to the gear (24), and further comprising a toggle-rocker device having an eccentric pin (27) connected to the ratchet wheel (25) and a rocker (28) hinged to the eccentric lever (5) of the axis (4).

3. A protective mask according to claim 1, characterised by that the device (6) for turning the eye plate (3) comprises a drum (30) connected one-side to the shell (1) and flexible thread (29) freely wound on the drum (30), where the thread (29) is hanged on its one end to the shell (1) by means of a spring (31), and its other end is connected to the under-mandible-lever (7), that is connected by a spring (32) to the shell (1), further comprises toggle-rocker device having eccentric pin (27) attached to the drum (30) and a rocker (28) flexibly connected to the eccentric lever (5) of the axis (4).

4. A protective mask according to claim 1, characterised by that the shell (1) has an anatomic shape following the characteristic outline of the projecting parts of the human face, comprising at least two parts, upper (20) and lower (21) flexibly connected each other in a way to be fixed in the desired position according to the size of the operator's face.

5. A protective mask according to claim 1, characterised by that the fixing device to the operator's head comprises clamps (9) supported by the concha cartilage, being the clamps (9) connected to both sides of the shell (1) by means of screw connectors (10) for tightening and fixing the shell (1) in a determined position, and in-between are connected by an elastic flexible joint (11) for tightening the mask to the face of the worker, and further comprises an adjustable rest (12) connected to the shell (1) in the area of the nostrils of the worker.

6. A protective mask according to claim 5, characterised by that it comprises a fixing clip (13) connected to the shell (1) having holders (14) for attachement to the upper part of the nose and springs (16) acting on the holders (14).

7. A protective mask according to claim 1 characterised by that the safety eye plate (3) is connected by screws (33) to stationarily fixed to the axis (4) supports (34).

8. A protective mask according to claim 1, characterised by that the safety eye plate (3) used for electric welding in its lower part is completely obscured, and in its upper part is medium obscured.
